# EUROPÄISCHE PATENTANMELDUNG

(11) **EP 1 284 107 A1**
(43) Veröffentlichungstag der Anmeldung: **19.02.2003**
(21) Anmeldenummer: 01119442.0
(22) Anmeldetag: 13.08.2001
(51) Int. Cl.: A41D 20/00, A41D 13/005, A61F 7/10

(54) **Kälte-und Wärmezellen, die Superabsorberpolymere enthalten und deren Verwendung**

(71) Anmelder: Yehia, Yousif, Dr. med., 89231 Neu-Ulm (DE)
(72) Erfinder: Yehia, Yousif, Dr. med., 89231 Neu-Ulm (DE)

(57) **Zusammenfassung**

Manche Erfindungen beschreiben Lösungen für die menschliche Körpererhitzung durch die Kühlwirkungen des Superabsorbers Polyacrylamid-Granulat. Das Risiko von Hautkontakt mit gesundheitsschädlichen Acrylamidmonomeren ist in diesem Fall sehr groß.
Eine andere Problematik ist, dass viele Naturheilstoffe nicht in anwendungsgerechter Form vorhanden sind. Für den normalen Verbraucher ist es auch nicht möglich, diese im Handel nicht vertriebenen Naturstoffe selbst herzustellen.

Die Erfindung betrifft eine Zelle, die beim Tragen auf der Haut Kühlung bzw. Wärmewirkung ausübt. Die Zelle kann auch Flüssigkeiten, die biologische Stoffe enthalten, speichern und dadurch durch die Haut biologische Wirkung oder Heilwirkung entfalten.
Eine Kälte- bzw. Wärmezelle besteht aus einem geschlossenen Hohlraum (Aktivzellraum) aus wasserdurchlässigem Material (1), wie Polyesterfaser oder Textilgewebe. Der Aktivzellraum wird mit gesundheitsunschädlichen Superabsorber, das sind Polymer Kristalle z.B. Polyacrylsäure oder Polyacrylat, gefüllt oder beschichtet (4).
Die Zelle kann zu modischen Kleidungsstücken wie Baseballcape, Westen, Stirnbändern, Nackenbändern, Visoren, Armbändern, Beinbändern, Brustbändern, modischen Kopfbedeckungen, Kältekompressen, Wärmekompressen, Gesichtsmasken, Schuhen oder Hausschuhen verarbeitet werden (8).

## Beschreibung

Die Erfindung betrifft eine Zelle, die beim Tragen auf der Haut Kühlung bzw. Wärmewirkung ausübt. Die Zelle kann Flüssigkeiten, die biologische Stoffe enthalten, speichern und dadurch durch die Haut biologische Wirkungen erzielen.

Wenn die Temperaturen in der Umgebung zu hoch sind, z.B. bei direkter Sonnenstrahlung oder in Räumen mit Wärmestrahlung, wird der menschliche Organismus überlastet. Der natürliche Abkühlungsmechanismus, der da durch entsteht, dass der Mensch Schweiß absondert, der dann auf der Hautoberfläche verdunstet, ist mit der Gefahr verbunden, dass der Körper dabei zu viel Flüssigkeit verliert und austrocknen kann. Bei solch hohen Temperaturen entsteht im Körper der sogenannte Hitzestau und die Gefahr, dass der menschliche Organismus keine Wärme mehr abgeben kann (Hitzeschlag).

Manche Erfindungen z.B.: (DE 20002723U1) beschreiben Lösungen für die menschliche Körpererhitzung durch die Kühlwirkungen des Superabsorbers Polyacrylamid-Granulat, der in Kleidungsstücke gefüllt wird und nach dem Absorbieren von Wasser getragen wird. Das Risiko von Hautkontakt mit gesundheitsschädlichen Acrylamidmonomeren ist in diesem Fall sehr groß.

Die Superabsorber sind Polymere, die die Fähigkeit haben, das 100- 500 fache Volumen Wasser oder wässrige Flüssigkeiten zu absorbieren und zu speichern, bis es verdunstet. Manche Superabsorber behalten diese Eigenschaft sogar nach Behandlung mit extremen Temperaturen (über 100°C bis unter-20°C) bei.
Polymerisiertes Polyacrylamid wird für viele industrielle Zwecke verwendet.
Die Reste von Acrylamidmonomeren (2-propenamid: CH2=CH-C(=O)-NH2), die nicht am Reaktionsprozess teilgenommen haben, sind in den kommerziellen Polyacrylamidpräparaten immer in niedriger Konzentration vorhanden. Obwohl polymerisiertes Polyacrylamid relativ unschädlich ist, können Acrylamidmonomere auch bei niedriger Konzentration schwere Nervenvergiftungen (Neurotoxizität) im zentralen und peripheralen Nervensystem, Erbmaterialschädigungen (Genomtoxizität), Augenschädigungen (Nystagmus), Allergien, Kontaktdermatitis, Ekzeme und Umweltbelastungen verursachen. (Weltgesundheitsorganisation, Distribution and Sales Service, 1211 Genf 27, Schweiz, 1985. S. 121 Bibl.), (C.G. Daughton, Govt Reports Announcements & Index (GRA&I), Issue 05,1989).
Acrylamid wird als Krebserreger (Karzinogen) eingestuft (National Institute for Occupational Safety and Health, 676 Columbia Parkway, Cincinnati, OH 45226, USA, 1991. 39p. ca. 110 ref.). Acrylamidmonomere werden schnell durch die Haut, durch Inhalation und durch den Magen absorbiert. 24 Stunden nach dem Hautkontakt konnte es im Blut als freies Monomer, proteingebundenes Monomer und in verschiedenen Körperorganen von Versuchstieren festgestellt werden (Abstracts of the XVIII International Congress on Occupational Health, Brighton, England, September 14-19, 1975, page 453, 1975).

Im Gegensatz zu Polyacrylamid gibt es andere Superabsorber z.B. Polyacrylsäure, Na-Polyacrylat und andere Polymere, die nicht auf Basis von Acrylamidmonomeren strukturiert sind. Diese werden als unschädlich betrachtet, da sie keine schädlichen Monomere enthalten. Polyacrylsäure und Polyacrylat wird für den menschlichen Gebrauch als unbedenklich eingestuft und z.B. im Hygienebereich zur Aufnahme von Körperflüssigkeiten wie Blut, Schweiß, Urin und anderen flüssigen Ausscheidungen verwendet, sowie in Babywindeln und hygienischen Frauenbinden. Weiterhin wird es für Wundabdeckungen, in Lebensmittelverpackungen und als Isolationsmittel, in Textilien für Bekleidungs- und Reinigungszwecke, sowie für die Verwendung im Bereich der Pflanzenzucht und als Depotmaterial verwendet (DE 19505709A1).

Viele Naturheilstoffe sind nicht in anwendungsgerechter Form vorhanden. Für den normalen Verbraucher ist es auch nicht möglich diese im Handel nicht vertriebenen Naturstoffe selbst herzustellen.

Der Erfindung liegt die Aufgabe zugrunde, die Wärme- und Kältespeicherungseigenschaft der in den unschädlichen Polymeren enthaltenen Flüssigkeiten zu nutzen. Die Kälte bzw. Wärmezellen können Flüssigkeiten absorbieren, die biologische Stoffe enthalten und somit durch die Haut eine biologische Wirkung erzielen.

Diese Aufgabe wird durch die in den Ansprüchen 1 bis 7 dargelegten technische Anleitung gelöst.

In den zugehörigen Zeichnungen wird die erfindungsgemäß gestaltete Kälte- bzw. Wärmezelle dargestellt:
Fig.1: Mit einem Teilraum in Draufsicht, geeignet für das Einsetzen z.B. in einem Nackenband oder Armband.
Fig. 2: Kälte- bzw. Wärmezelle nach Fig. 1 im Schnitt
Fig. 3: Mit zwei Teilräumen in Draufsicht, geeignet für das Einsetzen z.B. in einem Stirnband, Cape, Baseballcape, Weste, Visor, modische Kopfbedeckung, Armband oder Brustband.
Fig. 4: Mit mehreren Teilräumen in einer Ansicht, geeignet für das Einsetzen z.B. in Kälte- bzw. Wärmekompressen, Weste, modischen Kopfbedeckung, Cape, Brustband, Schuhe, Hausschuhe, Gesichtsmasken oder Sitzkissen.
Fig. 5: Eine erfindungsgemäß gestaltete Kälte- bzw. Wärmezelle, in Form eines Armbandes, von oben gesehen
Fig. 6: Armband nach Fig. 5 im Schnitt

Die Erfindung soll an den folgenden Ausführungsbeschreibungen näher erläutert werden:
1. Eine Kälte- bzw. Wärmezelle besteht aus einem geschlossenem Hohlraum (Aktivzellraum) aus wasserdurchlässigem Material (1), wie Polyesterfaser, Vlies oder Textilgewebe. Dieser Hohlraum kann in zwei (2) oder mehrere Teilräume (3) unterteilt werden, womit die Fläche für den Wärmeaustausch vergrößert wird. Dieser Aktivzellraum wird mit Superabsorber, unschädlichen Polymer-Kristallen z.B. Polyacrylsäure oder Polyacrylat gefüllt oder beschichtet (0,5% - 30% des Aktivzellraumvolumens) (4).
2. Ein Aktivzellraum kann direkt aus Textilgewebehohlraum (1) des Endprodukts bestehen und mit Polymerkristallen (4) gefüllt werden.
3. Eine andere Möglichkeit, um den Aktivzellraum konstruieren zu können, besteht aus einer Mischung aus 10 - 99% der Menge des oben genannten Polymers (4) und Watte oder Zellulosefasern (5). Damit werden die Polymere in dieser Mischung gleichmäßig verteilt und als weiche Füllung direkt in den Textilgewebehohlraum des Endprodukts oder in einem Hohlraum eines anderen wasserdurchlässigen Materials eingelegt.

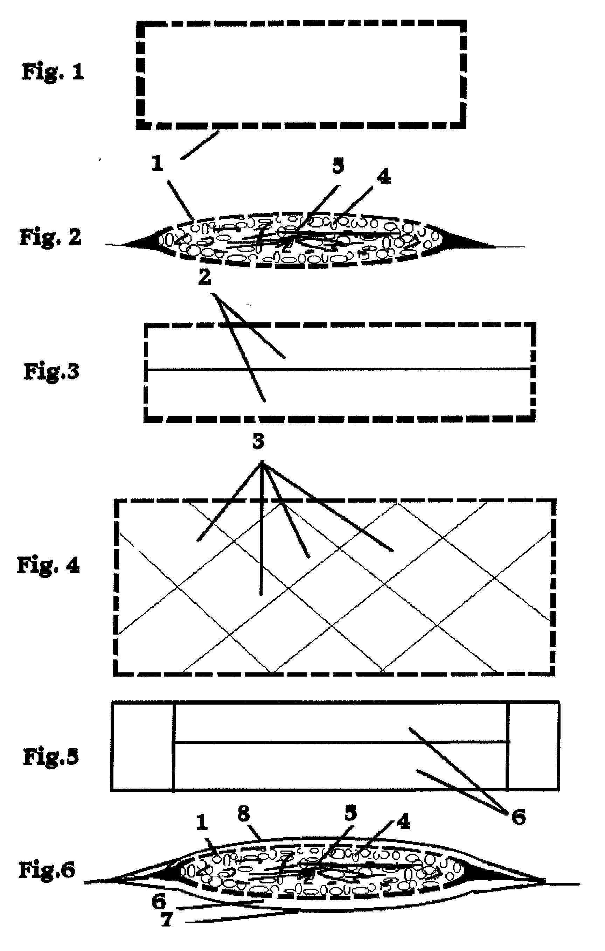

Dieser Aktivzellraum wird in textile Gewebe eingesetzt und als Endprodukt wie Westen, Baseballcapes, Stirnbänder, Nackenbänder, Armbänder, Kälte- bzw. Wärmekompressen, Sitzkissen, modische Kopfbedeckungen, Capes und Visoren verarbeitet. Dieser Aktivzellraum wird nach passendem Maß und Größe, je nachdem wie es die Verwendung des Endprodukts erfordert, angefertigt.
Die Zeichnungen (Fig.5) und (Fig.6) zeigen eine erfindungsgemäß gestaltete Kälte- bzw. Wärmezelle als Armband, die aus einem textilen Gewebe mit einer Leinenbindung besteht und doppelwandig ausgebildet ist. Die Kälte- bzw. Wärmezelle ist in den Hohlraum (6) zwischen den Textilbahnen (7) und (8) eingelagert.

Bei einer bestimmungsgemäßen Verwendung des Aktivzellraums zum Kühlen bzw. Wärmen muss das Endprodukt erst mit wässriger Flüssigkeit gesättigt werden. Hierzu wird das Endprodukt in ein Wasserbad gelegt, um zur Flüssigkeitsaufnahme für eine bestimmte Zeit zu verweilen.
Der Kühleffekt, der von dem erfindungsgemäßen Aktivzellraum bzw. Endprodukt ausgeht, kann je nach Verwendungszweck verstärkt werden, indem man es in den Kühlschrank oder in die Kühltruhe für eine bestimmte Zeit legt. Wenn das Endprodukt bei Raumtemperatur verwendet wird, dann basiert der Kühleffekt nur auf einer Verdunstung der im Polymer enthaltenen Flüssigkeiten.

Vorteilhaft ist, dass der erfindungsgemäße Aktivzellraum bzw. Das Endprodukt nicht durch die niedrige Temperatur beeinträchtigt wird und danach mehrmals wiederverwendet werden kann.
Die Polymere behalten die Flüssigkeit nach der Verschmelzung in sich. Deshalb entsteht kein Kondenswasser oder störende Wassertropfen. Der extra Kühlungseffekt vom Kühlschrank oder der Kühltruhe garantiert erhebliche Senkung der Körpertemperatur in kürzester Zeit. Damit ist es für Fieberbehandlung und als Erste Hilfe für Hitzeschlag geeignet. Nach einem Zeitraum von länger als zwei Tage und durch Befeuchtung der Füllung aus Polymer kann der Aktivzellraum ständig wieder eingesetzt werden. Die Verwendungsbereiche für den Kälteeffekt des erfindungsgemäßen Aktivzellraums sind vielfältig, z.B. als Kältekompresse bei akuten Beschwerden wie: Kopfschmerzen, Migräne, Fieber, Nasenbluten, Prellungen, Verstauchungen, Insektenstiche, Stoßverletzungen usw.

Der Aktivzellraum ist für Outdoor-Verwendungen sehr gut geeignet: z.B. Stirnbänder, Nackenbänder, Baseballcapes, modische Kopfbedeckungen, Visoren, Schuhen für lang Stehern und Setzern oder Autofahrern etc.

Der Wärmeeffekt des erfindungsgemäß gestalteten Aktivzellraums bzw. Endprodukts kann dadurch erzielt werden, indem die mit Wasser bzw. wässrigen Flüssigkeiten gefüllten Polymere für kurze Zeit (1-5 minuten) in die Mikrowelle oder heißes Wasser bzw. wässrigen Flüssigkeiten gelegt werden. Die Verwendungsbereiche für den Wärmeeffekt des erfindungsgemäßen Aktivzellraums als Wärmekompresse sind unter anderem: Verspannungen, Krämpfe, bei kalten Händen oder Füßen, chronische Beschwerden (Rheuma, Harnwege, Gelenke, etc.), Muskelhärte usw.

Der Entspannungseffekt und der Heilungseffekt kann verstärkt werden, indem man Duftstoffe oder ätherische Öle oder andere Stoffe, die biologische oder Heilwirkung haben, dem Wasser beifügt z.B. Menthol, Vitamine, Naturheilstoffe, pflanzliche Extrakte etc.
Das leichte Gewicht und das elegante Aussehen des Endproduktes ermöglichen vielfältigere Verwendungen im Vergleich zur herkömmlichen Kälte- oder Wärmekompresse.

## Patentansprüche

1. Kältezelle bzw. Wärmezelle, **dadurch gekennzeichnet, dass** sie aus einem Hohlraum (Aktivzellraum) (1)gefüllt oder beschichtet mit gesundheitsunschädlichen Polymeren, besteht.

2. Kältezelle bzw. Wärmezelle gemäß Anspruch 1, **dadurch gekennzeichnet, dass** der Hohlraum mit Flüssigkeiten absorbierenden Polymeren (4) oder einer Mischung die Flüssigkeiten absorbierende Polymere enthält (5), gefüllt oder beschichtet ist.

3. Kältezelle bzw. Wärmezelle gemäß Anspruch 1 und 2, **dadurch gekennzeichnet, dass** der Hohlraum aus einem wasserdurchlässigen Material besteht.

4. Kältezelle bzw. Wärmezelle gemäß Anspruch 1 bis 3, **dadurch gekennzeichnet, dass** der Hohlraum aus einem oder mehreren Teilräumen besteht (2, 3), wobei diese Teilräume mit Polymeren oder Mischungen, die Polymere enthalten, gefüllt sind.

5. Kältezelle bzw. Wärmezelle gemäß Anspruch 1 bis 4 **dadurch gekennzeichnet, dass** die Polymere bei Benutzungsbeginn mit Wasser oder anderen Flüssigkeiten gefüllt sind.

6. Kältezelle bzw. Wärmezelle gemäß Anspruch 1 bis 5 **dadurch gekennzeichnet, dass** die Zelle als Baseballcape, Weste, Stirnband, Nackenband, Visor, Armband, Beinband, Brustband, modische Kopfbedeckungen, Schuhe, Hausschuhe oder andere Kleidungsstücke oder, Kältekompresse, Wärmekompresse, Sitzkissen, Gesichtsmaske verarbeitet ist.

7. Kältezelle bzw. Wärmezelle gemäß Anspruch 1 bis 6 **dadurch gekennzeichnet, dass** die Polymere mit Duftstoffen oder andere Stoffen, die biologische Wirkungen haben, sowie Stoffen mit Heilwirkungen oder auch deren Lösungen behandelt sind.
